# EUROPÄISCHE PATENTSCHRIFT

(11) **EP 4 066 766 B1**
(45) Veröffentlichungstag und Bekanntmachung des Hinweises auf die Patenterteilung: **06.09.2023**
(21) Anmeldenummer: 21166329.9
(22) Anmeldetag: 31.03.2021
(51) Int. Cl.: A61B 18/12

(54) **ELEKTROMEDIZINISCHER LEISTUNGSGENERATOR**
ELECTROSURGICAL POWER GENERATOR
GÉNÉRATEUR ÉLECTROMÉDICAL DE PUISSANCE

(43) Veröffentlichungstag der Anmeldung: 05.10.2022
(73) Patentinhaber: Erbe Elektromedizin GmbH, 72072 Tübingen (DE)
(72) Erfinder: DORNHOF, Konstantin, 78194 Immendingen (DE)
(74) Vertreter: Rüger Abel Patentanwälte PartGmbB

(56) Entgegenhaltungen:
- DE-A1- 2 023 140
- DE-B1- 2 910 196
- US-A- 3 675 655
- US-A- 4 617 927

## Beschreibung

Die Erfindung betrifft einen elektromedizinischen Generator zur Speisung von einer oder mehreren Elektroden, die zur Durchführung elektrochirurgischer Maßnahmen an einem Patienten vorgesehen sind.

Zur Vermeidung neuromuskulärer Reizungen wird in der Elektrochirurgie typischerweise Wechselstrom mit einer Frequenz von deutlich oberhalb 100 kHz verwendet, der mit entsprechender Leistung bereitzustellen ist. Typischerweise liegt die Leistungsabgabe deutlich über 1 W und kann mehrere 100 W erreichen. Dazu sind entsprechende Leistungsgeneratoren erforderlich.

Elektrochirurgische Instrumente, Sonden oder dergleichen benötigen einen elektrochirurgischen Generator zur Speisung des Instruments mit hochfrequentem Wechselstrom. Dazu offenbart die DE 60 2004 009 293 T2 ein elektrochirurgisches System mit einem Generator, an den ein von dem Generator mit Hochfrequenzstrom zu speisendes Instrument anschließbar ist. In einer Ausführungsform weist das Instrument sowohl Koagulationselektroden als auch eine Schneidelektrode auf, die in schneller Folge alternierend beaufschlagt werden, um damit gleichzeitig zu arbeiten. Ein entsprechender elektronischer Umschalter ist dazu in dem Instrument selbst vorgesehen.

Die US 7 896 875 B2 und die US 2011/0112530 beschreiben jeweils ein HF-Instrument, dessen externer Generator von einer Batterie gespeist ist. Die US 9 155 585 B2 beschreibt außerdem einen batteriebetriebenen elektromedizinischen Generator mit fremdgesteuerten Transistoren. Aus der US 2015/0305798 geht ein Instrument mit eingebauter Batterie und eingebautem Generator hervor.

Weiter ist aus der EP 2 572 668 B1 wie auch aus der EP 2 572 669 B1 die Anwendung von Mikrowellen zur medizinischen Behandlung bekannt. Entsprechende Instrumente weisen an einem distalen Ende eines länglichen Schafts eine Mikrowellenantenne auf, die von einem in dem Instrument angeordneten Mikrowellenverstärker gespeist wird. Das Instrument ist über ein Kabel mit einem Mikrowellensignalgenerator verbunden, dessen Signal zu dem Mikrowellenverstärker geleitet wird. Bei einer modifizierten Ausführungsform ist der Mikrowellensignalgenerator in dem Handgriff des Instruments angeordnet. Ein Umschalter ermöglicht dann das Umschalten zwischen Mikrowellensignalen eines externen Signalgenerators und den Mikrowellensignal des internen Signalgenerators.

Auch aus der US 6 039 734 ist ein Instrument mit eingebautem Generator bekannt, das eine monopolare Elektrode zur Behandlung eines Patienten aufweist und den Stromkreis kapazitiv über den Behandler schließt. Die Arbeitsfrequenz ist größer als 5 MHz. Schließlich geht weiterer Stand der Technik aus der US 2017/238987 A1**,** der US 2017/202607 A1**,** der US 2017/079710 A1**,** der US 2016/0270841 A1**,** der US 2014/148803 A1**,** der DE 20 2008 001 365 U1 und der CA 2 286 835 A1 hervor.

Während Mikrowellengeneratoren Gewebe durch Einstrahlung von Mikrowellen erwärmen und beeinflussen, arbeiten hochfrequenzbetriebene chirurgische Instrumente mit wesentlich niedrigeren Frequenzen. Die Frequenz des zum Betrieb solcher Instrumente bereitgestellten Stroms liegt typischerweise bei einigen 100 kHz. Solche Instrumente arbeiten mit einem hochfrequenten Stromfluss durch das Gewebe und benötigen dazu immer zwei an dem Gewebe anliegende Elektrode. Die Instrumente dienen der Durchführung verschiedener Maßnahmen, die an den direkten Stromfluss durch das biologische Gewebe geknüpft sind, so zum Beispiel Schneiden, Koagulieren, Fusionieren, Abladieren oder dergleichen. Durch Form und Anwendung entsprechender Elektroden lassen dich die gewünschten chirurgischen Effekte zielgenau beeinflussen. Es kommen dabei verschiedene HF-Spannungen und -Ströme ebenso zur Anwendung wie verschiedene Modulationsformen, beispielsweise unmodulierte HF (CW - "Dauerstrich"), amplitudenmoduliert, beispielsweise gepulst mit oder ohne Pulsbreitenmodulation, und dergleichen mehr. Außerdem können über entsprechende Generatorausgangskennlinien Strom/Spannungs-Zusammenhänge festgelegt werden, die dem Operationserfolg förderlich sind.

Allerdings ist zum Betrieb eines solchen chirurgischen Instruments ein typischerweise externer chirurgischer Generator erforderlich, der die für das Instrument nötigen Modes bereitstellen muss und von dem die Hochfrequenzleistung typischerweise über ein Kabel zu dem Instrument übertragen wird. Die Modes unterscheiden sich z.B. in Spannung, Strom, Leistung, Modulation und vielem anderem mehr.

Die DE 29 01 153 A1**,** die US 2010/0137854 A1**,** die US 2011/0245826 A1 und die EP 1 599 146 B1 offenbaren solche Generatoren. Diese nutzen fremdgesteuerte Schalter zur Anregung eines oder mehrerer Schwingkreise. Ferner sind elektromedizinische Leistungsgeneratoren aus US4617927A und DE2910196B1 bekannt.

Außerhalb medizinischer Anwendungen, beispielsweise in der Telekommunikationstechnik, werden auch selbstschwingende Generatoren genutzt, wie sie beispielsweise aus der DE 197 80 481 D1**,** der DE 197 80 470 T1**,** der DE 197 19 440 C2 oder der DE 197 19 441 C2 bekannt sind. Diese Generatoren sind als spannungsgesteuerte Gegentaktoszillatoren für den Höchstfrequenzbereich von 1 bis 20 GHz konzipiert. Dabei soll insbesondere mit einer sehr niedrigen Betriebsspannung von beispielsweise lediglich 4,5 V gearbeitet werden. Die Schaltungen eignen sich für den Milliwatt-Bereich. Hingegen wird in der Elektrochirurgie mit erheblich größeren Leistungen und wesentlich höheren Spannungen gearbeitet. Dabei besteht die Gefahr der Spannungsüberlastung einzelner Bauteile.

Es ist Aufgabe der Erfindung, eine Generatorschaltung anzugeben, die sich für den Einsatz in der Elektrochirurgie eignet und bei einfachem Aufbau einen hohen Wirkungsgrad erreicht.

Diese Aufgabe wird mit einem Leistungsgenerator nach Anspruch 1 gelöst.

Der erfindungsgemäße Leistungsgenerator weist einen Schwingkreis auf, der zwischen den Ausgängen zweier Kaskodeschaltungen angeordnet ist. Die Kaskodeschaltungen sind rückkoppelnd (mitkoppelnd) miteinander verbunden und bilden somit zusammen mit dem Schwingkreis einen selbstschwingenden Generator. Dieses Konzept führt zu einem einfachen Generatoraufbau, bei dem die hochohmigen Ausgänge der Kaskodeschaltung die Güte des Schwingkreises nicht verschlechtern, wodurch sich eine gute Frequenzstabilität und eine hohe spektrale Reinheit der erzeugten Wechselspannung ergeben. Die beiden Kaskodeschaltungen arbeiten im Gegentakt, wobei durch die wechselseitige Mitkopplung Schaltverluste in den beiden Kaskodeschaltungen minimiert werden. Die Umschaltzeitpunkte der einzelnen Transistoren werden durch die Schwingung des Parallelschwingkreises ideal vorgegeben. Durch die Minimierung der Schaltverluste eignet sich das Konzept insbesondere für Generatoren, die eine hohe, zum Einsatz in der Elektrochirurgie geeignete Leistung abgeben.

Die Kaskodeschaltung weisen jeweils einen Eingangstransistor in Emitter oder Drain-Schaltung und einen Ausgangstransistor in Basis- oder Gate-Schaltung auf. Die in Basis- oder Gate-Schaltung betriebenen Ausganstransistoren können an ihren Ausgangselektroden (Kollektor oder Drain) die an dem Schwingkreis auftretenden hohen Spannungen ohne Gefahr für den jeweiligen Ausgangstransistor ertragen. Die erfindungsgemäße Generatorschaltung eignet sich auch deswegen insbesondere für die Anwendung in der Elektrochirurgie.

Die Steuerelektrode (Basis oder Gate) des Eingangstransistors einer der Kaskodeschaltungen ist mit der Ausgangselektrode (Kollektor oder Drain) des Eingangstransistors der anderen Kaskodeschaltung verbunden. Die Ausgangselektroden der Eingangstransistoren sind mit dem Stromeingängen (Emitter oder Source) der Ausgangstransistoren der Kaskodeschaltungen verbunden. Der Verbindungspunkt zwischen der Ausgangselektrode des Eingangstransistors und dem Stromeingang des Ausgangstransistors bildet einen Zwischenabgriff. An diesem Zwischenabgriff ist auch bei hohen Betriebsspannungen nur eine geringe Spannung (zwischen 0 und z.B. 20 V) zu verzeichnen, sodass die Eingangstransistoren an ihren Steuerelektroden (Basis oder Gate) keinen hohen Spannungen ausgesetzt sind. Damit ist auch hier der zum Schutz der Steuerelektroden zu treibende technische Aufwand minimiert. Zusätzliche Bauelemente, wie Dioden oder dergleichen zum Schutz vor Überspannungen, können entfallen. Die kapazitive Belastung der Steuerelektroden ist minimiert, wodurch Schaltflanken mit maximaler Steilheit wirksam werden können und Schaltverluste wiederum gesenkt werden.

Die Steuerelektroden der Eingangstransistoren können mit dem Zwischenabgriff insbesondere kapazitiv verbunden werden. Außerdem können Sie untereinander resistiv verbunden sein, um einen Potentialdrift zu unterbinden und symmetrisches Schalten zwischen den beiden Kaskodeschaltungen zu erzwingen.

Die Steuerelektroden der Ausgangstransistoren sind vorzugsweise mit einem festen Potential, z.B. Masse oder einer Gleichspannung verbunden. Die Gleichspannung kann aus der Versorgungsspannung abgeleitet sein.

Wenn der Transistor normalleitend ist, wie es z.B. bei GaN-Transistoren oft der Fall ist, können die Steuerelektroden direkt gegen Masse geschaltet werden.

Insbesondere kann dies durch einen linearen oder nichtlinearen Spannungsteiler erfolgen, der beispielsweise eine Z-Diode oder ein anderes eine in Bezug auf Bezugspotential (Masse) feste Spannung erzeugendes Element sein kann. Vorzugsweise sind die Steuerelektroden der Ausgangstransistoren kapazitiv mit Bezugspotential (Masse) verbunden.

Der an die Ausgänge der Kaskodeschaltungen angeschlossene Parallelschwingkreis weist eine Spule mit einer Mittelanzapfung auf, die, vorzugsweise über eine Drossel, mit einer Gleichspannungsquelle verbunden ist. Die Gleichspannungsquelle kann beispielsweise eine Gleichspannung von 100 V, 150 V, 200 V oder mehr bereitstellen. Die zu den Elektroden eines Instruments zu leitende Spannung kann von dieser Spule transformatorisch abgegriffen werden.

Es ist möglich, die Gleichspannung zu modulieren, beispielsweise indem diese zwischen einem Minimalwert und einem Maximalwert alterniert. Die entsprechend ausgebildet Versorgungseinrichtung kann außerhalb eines Instruments beispielsweise als stationäres Gerät ausgebildet sein. Der Generator ist schaltungsmäßig einfach aufgebaut und kann aufgrund seiner geringen Verlustleistung direkt im Instrument angeordnet sein. Verschiedene Betriebsarten des Instruments, beispielsweise mit unmodulierter Hochfrequenz oder auch mit gepulster oder anderweitig modulierter Hochfrequenz, können ausgewählt werden, indem die Versorgungseinrichtung entweder eine konstante Gleichspannung oder eine zwischen verschiedenen Gleichspannungswerten alternierende oder um einen Mittelwert schwingende Gleichspannung liefert.

Mit dem erfindungsgemäßen Konzept lassen sich HF-Leistungsgeneratoren für den elektrochirurgischen Einsatz mit äußerst hohem Wirkungsgrad auch bei Frequenzen oberhalb mehrerer 100 kHz, z.B. oberhalb 500 kHz erstellen. Es können mit geringsten Schaltverlusten Frequenzen von bis zu 4 MHz und darüber erzeugt werden, wobei eine hohe Frequenzstabilität und gute spektrale Reinheit erzielt werden.

Einzelheiten der Erfindung ergeben sich aus den Figuren, der Zeichnung sowie der zugehörigen Beschreibung und Ansprüchen. Es zeigen:
Figur 1 ein an eine Versorgungseinrichtung angeschlossenes Instrument zum elektromedizinischen Einsatz,
Figur 2 einen Leistungsgenerator zur Speisung der Elektroden des Instruments nach Figur 1, in Prinzipdarstellung,
Figur 3 den Leistungsgenerator nach Figur 2, in detaillierter Schaltung,
Figur 4 von dem Leistungsgenerator nach Figur 3 abgegebene Spannungen in verschiedenen Arbeitsmoden als Diagramm,
Figur 5 die Versorgungseinrichtung und den Leistungsgenerator in schematisierter Blockdarstellung.

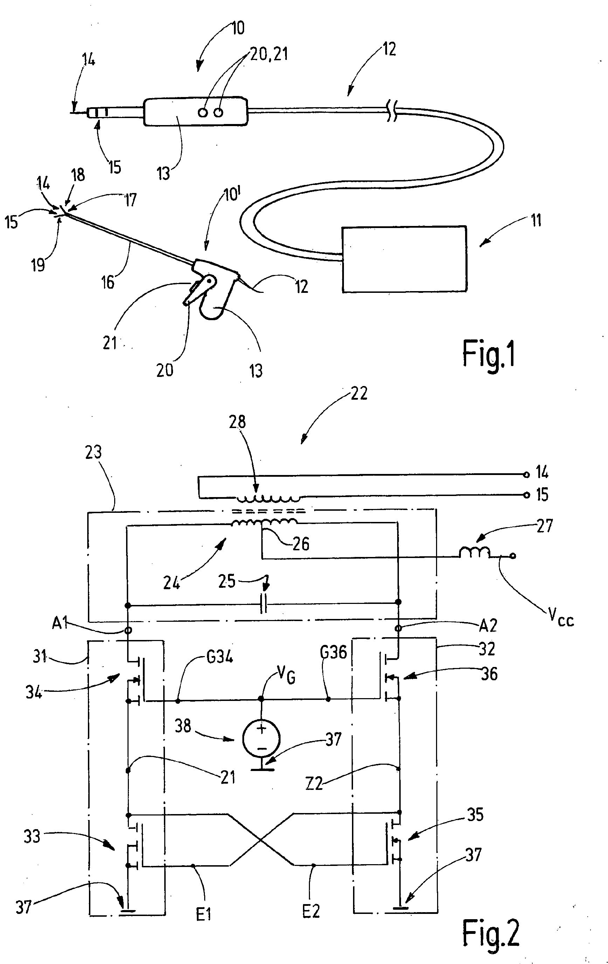

In Figur 1 sind ein elektrochirurgisches Instrument 10 und eine Versorgungseinrichtung 11 veranschaulicht, die untereinander durch ein Kabel 12 verbunden oder verbindbar sind. Die Versorgungseinrichtung 11 ist darauf eingerichtet, elektrische Leistung über das Kabel 12 an das Instrument 10 zu liefern. Das Instrument 10 kann ein für den offenchirurgischen Einsatz vorgesehenes Instrument oder ein für den laparoskopischen oder anderweitigen Einsatz vorgesehenes Instrument 10' sein. Figur 1 veranschaulicht dazu ein offenchirurgisches und ein laparoskopisches Instrument.

Bei dem vorliegenden Ausführungsbeispiel ist das Instrument 10 mit Gleichspannung betrieben. Die Versorgungseinrichtung 11 stellt für das Instrument 10 eine Gleichspannung, eine ein- und ausschaltbare Gleichspannung, eine pulsierende Gleichspannung, eine um einen Mittelwert schwingende Gleichspannung oder auch einen Gleichstrom bereit. Außerdem kann die Versorgungseinrichtung 11 dazu eingerichtet sein, eine Wechselspannung abzugeben, die dann aber in dem Instrument 10 zunächst gleichgerichtet wird, um damit einen Leistungsgenerator zu betrieben.

Das Instrument 10 kann ein Instrument mit einem Handgriff 13 sein, an dem eine Elektrode 14, beispielsweise eine Spatelelektrode, eine Nadelelektrode oder dergleichen und gegebenenfalls auch eine Gegenelektrode 15 vorgesehen sind. Auch das für den laperoskopischen Einsatz vorgesehene Instrument 10` weist einen Handgriff 13 auf, von dem sich ein langer schlanker Schaft 16 weg erstreckt. An dem distalen Ende des Schafts 16 ist ein Werkzeug 17, beispielsweise in Gestalt einer Kauterisierungszange, angeordnet, deren Branchen 18, 19 die Elektroden 14, 15 traten. An dem Handgriff 13 können Bedienelemente 20, 21 zum mechanischen Betätigen des Werkzeugs 17 und/oder zur elektrischen Aktivierung der Elektroden 14, 15 vorgesehen sein.

Zur Speisung der Elektroden 14, 15 dient ein Leistungsgenerator 22, der in Figur 2 veranschaulicht ist. Dieser Leistungsgenerator 22 ist, wie es bevorzugt wird, in dem Instrument 10, 10', dort vorzugsweise in dem Handgriff 13 angeordnet. Der Leistungsgenerator 22 kann aber bei anderen Ausführungsformen prinzipiell auch an anderer Stelle angeordnet sein, z.B. im Verlauf des Kabels 12, in einem Stecker des Kabels 12 oder in der Versorgungseinrichtung 11 selbst. Bei dieser letztgenannten Ausführungsform führt das Kabel 12, anders als bei den sonstigen Ausführungsformen, hochfrequente Spannung und hochfrequenten Strom. Das Instrument 10 kann auch als monopolares Instrument ausgebildet sein. Es weist dann eine zur Behandlung vorzugsweise am distalen Ende angeordnete Elektrode und eine großflächige über einen Neutralleiter mit dem Instrument verbundene Neutralelektrode auf. Diese kann als Klebeelektrode ausgebildet sein, die an dem Patienten zur Stromrückführung angebracht wird. Bei solchen Ausführungsformen kann der Generator auch in oder an der Neutralelektrode, in dem Neutralleiter oder an jedem anderen vorgenannten Ort angebracht sein.

Der Leistungsgenerator 22 ist in Figur 2 zur Erläuterung seiner Baugruppen schematisiert veranschaulicht. Zu dem Leistungsgenerator 22 gehört ein Schwingkreis 23, der zum Beispiel aus einer Spule 24 und einem Kondensator 25 besteht, die zueinander parallel geschaltet sind. Dieser Schwingkreis 23 ist die frequenzbestimmende Baugruppe des Leistungsgenerators 22. Die Spule 24 ist vorzugsweise mit einer Mittelanzapfung 26 versehen, die gegebenenfalls über eine Drossel 27 an eine Betriebsspannung VCC angeschlossen ist. Die Betriebsspannung VCC kann über das Kabel 12 von der Versorgungseinrichtung 11 geliefert werden. Die Mittelanzapfung 26 kann genau mittig angeordnet sein, so dass die zu ihren beiden vorhandenen Spulenhälften exakt die gleiche Induktivität aufweisen. Die Mittelanzapfung 26 kann aber auch etwas "außermittig" angeordnet sein, so dass die beiden Spulenhälften (etwas) unterschiedliche Induktivitäten aufweisen. Dies kann das Anschwingen des Leistungsgenerators 22 erleichtern. Die kann den Betrieb im gepulsten Mode erleichtern.

Die Spule 26 kann in magnetischer Kopplung mit einer Auskoppelspule 28 stehen und mit dieser einen Transformator bilden. Das Windungsverhältnis der Spule 24 zu der Auskoppelspule 28 ist den Erfordernissen des elektrochirurgischen Behandlungsvorgangs entsprechend festgelegt und kann somit sowohl größer als auch kleiner als 1 sein. Auch der zwischen Null und Eins liegende Kopplungsfaktor zwischen der Spule 26 und der Auskoppelspule 28 kann den Erfordernissen des elektrochirurgischen Behandlungsvorgangs entsprechend festgelegt sein. Die Auskoppelspule bildet zusammen mit dem zwischen den Elektroden 14, 15 gefassten Gewebe vorzugsweise einen galvanischen Stromkreis ohne Verzweigungen.

Der (Parallel-)Schwingkreis 23 ist an Ausgänge 29, 30 zweier Kaskodeschaltungen 31, 32 angeschlossen. Zu der ersten Kaskodeschaltung 31 gehört ein erster Eingangstransistor 33 und ein erster Ausgangstransistor 34. Zu der zweiten Kaskodeschaltung 32 gehören ein zweiter Eingangstransistor 35 und ein zweiter Ausgangstransistor 36. Die Transistoren 33 bis 36 sind Schalttransistoren, vorzugsweise Feldeffekt-Transistoren. Prinzipiell können jedoch auch Bipolartransistoren, IGBTs oder andere gesteuerte Schalter Anwendung finden. Die nachfolgende Beschreibung gilt für Feldeffektransistoren und bipolare Transistoren entsprechend, mit der Maßgabe, dass anstelle von Gate, Sorce und Drain Basis, Emitter und Kollektor stehen. Das vorgestellte Schaltungsprinzip eignet sich sowohl für p- oder n-Feldeffekttransistoren vom Anreicherungsty oder vom Verarmungstyp sowie für pnp- oder npn-Bipolartransistoren.

Die erste Kaskodeschaltung 31 weist einen ersten Eingang E1, einen Zwischenabgriff Z1 und einen ersten Ausgang A1 auf. Entsprechend weist die zweite Kaskodeschaltung 32 einen zweiten Eingang E2, einen zweiten Zwischenabgriff Z2 und den Ausgang A2 auf. Der erste Eingang E1 wird durch die Steuerelektrode des ersten Eingangstransistors 33 gebildet. Ist der erste Eingangstransistor 33 ein FeldeffektTransistor oder ein IGBT ist die Steuerelektrode das Gate. Im Falle eines Bipolartransistors ist die Steuerelektrode seine Basis.

Der zweite Eingangstransistor 35 weist ebenfalls eine entsprechende Steuerelektrode z.B. ein Gate oder eine Basis auf, die den zweiten Eingang E2 bildet.

Der erste Eingangstransistor 33 weist einen Source-Anschluss auf, der mit Bezugspotential, z.B. Masse 37 verbunden ist. Im Falle der Verwendung von Bipolartransistoren ist der Emitter dieses ersten Eingangstransistors 33 mit Masse 37 verbunden. Entsprechendes gilt für den zweiten Eingangstransistor 35.

Die Eingangstransistoren 33, 35 weisen jeweils eine Ausgangselektrode (Drain oder Emitter) auf, die den Zwischenabgriff Z1 bzw. Z2 bildet und jeweils mit dem Stromeingang des Ausgangstransistors 34, 36 verbunden ist. Der Stromeingang des Ausgangstransistors 34, 36 ist jeweils dessen Source-Elektrode (bzw. Emitter, falls Bipolartechnik zur Anwendung kommt). Die Steuerelektroden der beiden Ausgangstransistoren 34, 36 sind entweder unabhängig voneinander oder gemeinsam mit einem festen Potential verbunden. Dazu können sie mit einer Konstantspannungsquelle 38 verbunden sein, die ein im Wesentlichen konstantes Potential oberhalb des Bezugspotentials 37 liefert. Die Steuerelektroden der Ausgangstransistoren 34, 36 sind deren Gate-Elektroden oder Basen, im Falle von Bipolartransistoren. Werden normal leitende Transistoren verwendet, kann das Bezugspotential auch 0 V (Masse) sein.

Die Drain- oder Kollektorelektroden der beiden Ausgangstransistoren 34, 36 bilden die Ausgänge A1, A2 der beiden Kaskodeschaltungen 31, 32.

Der Eingang E1 ist mit dem Zwischenabgriff Z2 verbunden. Der Eingang E2 ist mit dem Zwischenabgriff Z1 verbunden. Damit bilden die Eingangstransistoren 33, 35 miteinander eine Kippschaltung, die allein nicht schwingfähig ist, sondern vielmehr bistabilen Charakter aufweist. Die nachgeordneten Ausgangstransistoren 34, 36 in Gate-Schaltung sind zugleich Last und Taktgeber für die aus den Eingangstransistoren 33, 35 gebildete Kippschaltung.

Der insoweit beschriebene Leistungsgenerator 22 arbeitet wie folgt:
Zur Inbetriebsetzung des Leistungsgenerators 22 wird diese an der Mittelanzapfung 26 mit einer Betriebsspannung VCC versorgt. Diese beträgt beispielsweise +150 V gegenüber Bezugspotential 37 (Masse). Abweichende Spannungswerte z.B. 10 V oder jeder dazwischen liegende Wert kann Anwendung finden.

Nach dem Anschwingen des Leistungsgenerators 22 liegt an den Ausgängen A1 und A2 eine um 180° phasenversetzte Schwingung symmetrisch zu der Betriebsspannung VCC an. Die dadurch die Ausgangstransistoren 34, 36 periodisch durchfließenden Ströme sind Taktgeber für die aus den Eingangstransistoren 33, 35 bestehende Kippschaltung, die somit im Rhythmus der Schwingung des Schwingkreises 23 hin und her schaltet. Mit anderen Worten, die Eingangstransistoren 33, 35 schalten abwechselnd ein und aus (werden abwechselnd leitend oder gesperrt). Die an den Zwischenabgriffen Z1, Z2 anliegende zumindest näherungsweise rechteckförmigen Spannungen werden von den Ausgangstransistoren 34, 36 verstärkt und unterhalten somit die Schwingung des Schwingkreises 23, auch wenn dieser über die Auskoppelspule 28 Strom an die Elektroden 14, 15 liefert. Dieser Generator erweist sich als frequenzstabil wobei er eine Schwingung von großer spektraler Reinheit erzeugen kann.

Je nach Höhe der an den Leistungsgenerator 22 angelegten Betriebsspannung VCC und/oder je nach Höhe des Windungsverhältnisses zwischen der Spule 24 und der Auskoppelspule 28 liegt an den Elektroden 14, 15 eine zur Koagulation, zur Dissektion, zur Ablation, zur Fulguration oder zur Gewebefusion oder zu anderen Zwecken geeignete Spannung an. Der Spannungsbereich kann dabei von unter 100 V bis zu mehreren 100 V gehen. Es sind Leistungen im mittleren Leistungsbereich zwischen 1 und 100 W wie auch Leistungen im oberen Leistungsbereich von 100 W bis mehrere 100 W erzielbar. Die Transistoren 33 bis 36 arbeiten dabei in reinen Schaltbetrieb mit minimalen Schaltverlusten infolge phasenrichtiger und präziser Umschaltung. Die Transistoren 33 bis 36 schalten im strom- und spannungslosen Zustand. Der Leistungsgenerator 22 ist somit sehr stark miniaturisierbar. Die Verlustleistung liegt im Bereich weniger Watt oder Bruchteilen von Watt. Dies gilt insbesondere für Frequenzen zwischen 100 kHz und 5 MHz.

Durch die Versorgung des Leistungsgenerators mit Gleichspannung ist es besonders vorteilhaft, wenn der Leistungsgenerator 22 in dem Instrument 10, 10' angeordnet ist. Die in dem Label 12 vorhandenen elektrischen Leitungen stehen unter Gleichspannung. Mittels der Drossel 27 kann gegebenenfalls in Verbindung mit einem nicht weiter veranschaulichten Pufferkondensator der in dem Kabel 12 fließende Strom soweit vergleichmäßigt werden, dass die überlagerte Welligkeit vernachlässigbar ist. Dies ermöglicht die Verwendung nicht oder nur schwach geschirmter Kabel, die entsprechend flexibel sind und somit die Handhabung des Instruments 10 erleichtern.

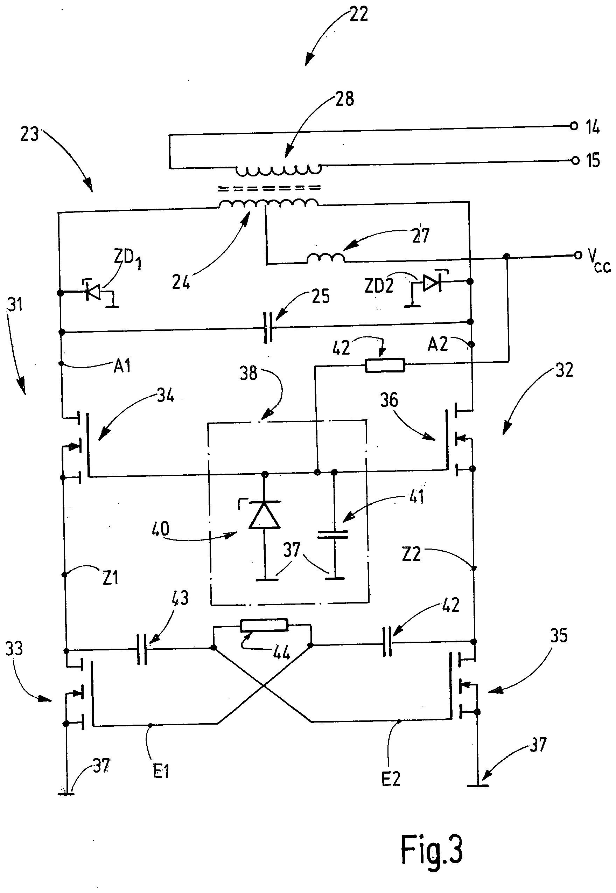

Figur 3 veranschaulicht den Leistungsgenerator 22 nochmals in etwas detaillierterer Form. Zur Beschreibung desselben gelten die bereits eingeführten Bezugszeichen und Erläuterungen entsprechend.

Die Konstantspannungsquelle 38 wird im vorliegenden Ausführungsbeispiel durch eine Z-Diode 40 gebildet, deren Kathode mit den Steuerelektroden der Ausgangstransistoren 34, 35 und deren Anode mit dem Bezugspotential (Masse) 37 verbunden ist. Der Z-Diode 40 kann ein Kondensator 41 parallel geschaltet sein, um das an der Kathode der Z-Diode 40 anstehende Signal wechselspannungsmäßig mit Masse zu verbinden. Ein Widerstand 42 kann die Konstantspannungsquelle 38 mit Strom versorgen und verbindet entsprechend den Betriebsspannungsanschluss VCC mit den Steuerelektroden (Gates) der Transistoren 32, 34 und der Konstantspannungsquelle 38. Z-Dioden ZD1 und ZD2 können die Ausgänge A1 und A2 mit Masse (Bezugspotential 27) verbinden und somit gegen Überspannungen absichern.

Weiter kann die Verbindung zwischen der Eingangselektrode E1 und dem Zwischenabgriff Z2 über einen Koppelkondensator 42 erbracht werden. Weiter kann ein Koppelkondensator 43 die Verbindung zwischen dem Zwischenabgriff Z1 und der Steuerelektrode E2 erbringen. Die Steuerelektroden oder Steuereingänge E1, E2 können über einen Widerstand 44 gleichstrommäßig miteinander verbunden sein, um an den Steuerelektroden E1 und E2 im zeitlichen Mittel übereinstimmende Potentiale zu erzeugen.

Auch bei diesem Leistungsgenerator 22 ist der Schwingkreis 23 das frequenzbestimmende Element. Die Koppelkondensatoren 42, 43 sind hingegen nicht frequenzbestimmen. Sie sind vielmehr so groß ausgelegt, dass der Leistungsgenerator 22 ohne den frequenzbestimmenden Schwingkreis nicht oder nur mit wesentlich geringerer Frequenz arbeiten würde.

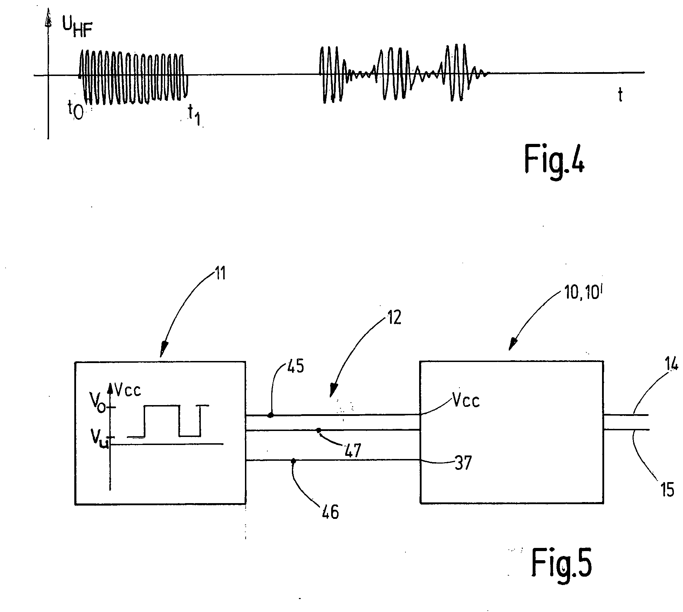

Bei der vorigen Beschreibung ist davon ausgegangen worden, dass die Versorgungseinrichtung 11 das Instrument 10 konstant mit Gleichspannung und Gleichstrom versorgt. Die Bedienelemente 20, 21 können als elektrische Schalter dienen, um den Leistungsgenerator 22 gezielt mit der Versorgungseinrichtung 12 zu verbinden oder von dieser zu trennen. Damit lässt sich das Instrument 10, 10' aktivieren der deaktivieren. Figur 4 veranschaulicht dazu den zeitlichen Verlauf der Spannung UHF von einem Zeitpunkt t0, bei dem der Leistungsgenerator 22 eingeschaltet ist, bis zu einem Zeitpunkt t1, zu dem der Leistungsgenerator 22 ausgeschaltet wird. Die Zeitspanne zwischen den Zeitpunkten t0 und t1 entspricht der Aktivierung des Instruments 10, 10`. Die HF-Spannung UHF liegt während dieser Aktivierung an den Elektroden 14, 15 im Wesentlichen unvermindert und ununterbrochen an.

Es ist jedoch auch möglich, die Versorgungseinrichtung 11 zur Abgabe einer pulsierenden (oder anderweitig modulierten) Gelichspannung einzurichten. Diese kann beispielsweise zwischen einem Spannungswert von 150 V und einem Spannungswert von 10 V schwanken, wie in Figur 5 links für die Versorgungseinrichtung 11 veranschaulicht ist. Der obere Spannungswert Vo wie auch der unter Spannungswert Vu gemäß dem Spannungsverlauf in Figur 5 können fest auf beispielsweise 150 V und 10 V festgelegt sein. Es können auch andere Spannungswerte genutzt werden. Auch können der obere Spannungswert Vo und/oder der untere Spannungswert Vu einstellbar sein. Im vorliegenden Ausführungsbeispiel ist die Versorgungseinrichtung 11 zum umschaltenden Alternieren zwischen dem oberen Spannungswert Vo und dem unteren Spannungswert Vu eingerichtet, es ergibt sich eine Reckteckkurvenform. Jedoch kann die Betriebsspannung VCC auch einen anderen Zeitverlauf aufweisen, beispielsweise eine Sägezahnverlauf, einen Dreieckspannungsverlauf, einen Trapezspannungsverlauf oder einen welligen Spannungsverlauf (z.B. Sinusform + Gleichanteil). Für die in Figur 5 dargestellte Rechteckform der Betriebsspannung VCC ergibt sich der in Figur 4 veranschaulichte HF-spannungsverlauf mit einer schmalbandigen Modulation der Hochfrequenzspannung.

Figur 5 veranschaulicht weiter das Zusammenspiel zwischen dem Instrument 10, 10' und der Versorgungseinrichtung 11 über das Kabel 12. Dieses enthält zumindest eine Betriebsspannung VCC führende Ader 45, sowie eine Bezugspotential 37, d.h. masseführende Ader 46. Zusätzlich können ein der mehrere Adern 47 vorgesehen sein, die zur Übertragung von Steuersignalen zwischen dem Instrument 10, 10' und der Versorgungseinrichtung 11 dienen. Damit wird es möglich, an dem Instrument 10, 10' zum Beispiel die Höhe der Betriebsspannung VCC, deren Modulation (Modulationsform, Modulationstiefe, Modulationsfrequenz usw.) einzustellen.

Der erfindungsgemäße Leistungsgenerator 22 ist selbstoszillierend aufgebaut. Er umfasst zwei Kaskodeschaltungen 31, 32, deren Ausgänge A1, A2 mit einem Parallelschwingkreis 23 verbunden sind, um diesen im Gegentakt anzuregen. Die Eingangstransistoren 33, 35 der Kaskodeschaltungen 31, 32 sind kreuzgekoppelt, während die Steuerelektroden der Ausgangstransistoren 34, 36 auf festem Potential liegen. Der Leistungsoszillator 22 ist selbstgesteuert, sodass die Transistoren 33 bis 36 geringste Schaltverluste aufweisen.

### Bezugszeichen:

- 10, 10`: Instrument
- 11: Versorgungseinrichtung
- 12: Kabel
- 13: Handgriff
- 14, 15: Elektroden
- 16: Schaft
- 17: Werkzeug
- 18, 19: Branchen
- 20, 21: Bedienelemente
- 22: Leistungsgenerator
- 23: Schwingkreis
- 24: Spule
- 25: Kondensator
- 26: Mittelanzapfung
- 27: Drossel
- V_{cc}: Betriebsspannung
- 28: Auskoppelspule
- A1, A2: Ausgänge der Kaskodeschaltungen
- 31: erste Kaskodeschaltung
- 32: zweite Kaskodeschaltung
- 33: erster Eingangstransistor
- 34: erster Ausgangstransistor
- 35: zweiter Eingangstransistor
- 36: zweiter Ausgangstransistor
- G₃₄: Steuerelektrode des ersten Ausgangstransistors 34
- G₃₆: Steuerelektrode des zweiten Ausgangstransistors
- 36 V_{G}: Gleichspannung
- 37: Bezugspotential
- 38: Konstantspannungsquelle
- 40: Z-Diode
- 41: Kondensator
- 42, 43: Koppelkondensator
- 44: Widerstand
- 45, 46: Adern des Kabels 12
- 47: Weitere Ader des Kabels 12 für Steuersignale
- ZD1, ZD2: Z-Dioden

## Patentansprüche

1. Elektromedizinischer Leistungsgenerator (22), insbesondere zur Erzeugung einer Hochfrequenzspannung (U_{HF}) zur Behandlung von biologischem Gewebe,
mit einem Schwingkreis (23), der mindesens einen Kondesator (25) und mindestens eine Spule (24) aufweist, die zueinander parallel geschaltet sind, um einen Parallelschwingkreis zu bilden,
mit einer ersten Kaskodeschaltung (31), die einen ersten Eingangstransistor (33) und einen ersten Ausgangstransistor (34) aufweist,
mit einer zweiten Kaskodeschaltung (32), die einen zweiten Eingangstransistor (35) und einen zweiten Ausgangstransistor (36) aufweist,
wobei die die beiden Ausgangstransistoren (34, 36) mit dem Schwingkreis (23) und die beiden Eingangstransistoren (33, 35) untereinander verbunden sind.

2. Leistungsgenerator nach Anspruch 1, **dadurch gekennzeichnet,**
**dass** die erste Kaskodeschaltung (31) einen ersten Eingang (E1), einen ersten Ausgang (A1) und einen ersten Zwischenabgriff (Z1) aufweist,
**dass** die die zweite Kaskodeschaltung (32) einen zweiten Eingang (E2), einen zweiten Ausgang (A2) und einen zweiten Zwischenabgriff (Z2) aufweist,
wobei die Ausgänge (A1, A2) der Kaskodeschaltungen (31, 32) mit dem Parallelschwingkreis (23) verbunden sind und
wobei der erste Zwischenabgriff (Z1) mit dem zweiten Eingang (E2) und der zweite Zwischenabgriff (Z2) mit dem ersten Eingang (E1) verbunden sind.

3. Leistungsgenerator nach Anspruch 2, **dadurch gekennzeichnet, dass** die Eingänge (E1, E2) mit den Zwischenabgriffen (Z2, Z1) kapazitiv verbunden sind.

4. Leistungsgenerator nach einem der vorstehenden Ansprüche, **dadurch gekennzeichnet, dass** die beiden Ausgangstransistoren (34, 36) jeweils eine Steuerelektrode (G₃₄, G₃₆) aufweisen und dass die Steuerelektroden (G₃₄, G₃₆) mit einer Gleichspannung (V_{G}) verbunden sind.

5. Leistungsgenerator nach einem der vorstehenden Ansprüche, **dadurch gekennzeichnet, dass** die Eingangstransistoren (33, 35) Feldeffekttransistoren sind und jeweils ein Source aufweisen, das mit einem Bezugspotential (37) verbunden ist.

6. Leistungsgenerator nach Anspruch 5, **dadurch gekennzeichnet, dass** die Eingangstransistoren (33, 35) jeweils ein Drain aufweisen, das dem Zwischenabgriff (Z1, Z2) verbunden ist

7. Leistungsgenerator nach Anspruch 5 oder 6, **dadurch gekennzeichnet, dass** die Eingangstransistoren (33, 35)jeweils ein Gate als Steuerelektrode aufweisen, wobei die Gates die Eingänge (E1, E2) der beiden Kaskodeschaltungen (31, 32) sind.

8. Leistungsgenerator nach einem der vorstehenden Ansprüche, **dadurch gekennzeichnet, dass** die Ausgangstransistoren (34, 36) Feldeffekttransistoren sind.

9. Leistungsgenerator nach Anspruch 8, **dadurch gekennzeichnet, dass** die Ausgangstransistoren (34, 36) jeweils ein Source aufweisen, das mit dem Zwischenabgriff (Z1, Z2) verbunden ist.

10. Leistungsgenerator nach einem der vorstehenden Ansprüche, **dadurch gekennzeichnet, dass** die Spule (24) einen Mittelabgriff (26) aufweist, der mit einer Versorgungsspannungsquelle (Vcc) verbunden ist.

11. Leistungsgenerator nach einem der vorstehenden Ansprüche, **dadurch gekennzeichnet, dass** die Spule (24) in magnetischer Kopplung mit einer Auskoppelspule (28) angeordnet ist.

12. Leistungsgenerator nach Anspruch 9, **dadurch gekennzeichnet, dass** die Auskoppelspule (28) mit mindestens einer Elektrode (14, 15) verbunden ist.

13. Instrument (10, 10`), in dem der Leistungsgenerator nach einem der vorstehenden Ansprüche angeordnet ist.

14. Einrichtung mit einem Leistungsgenerator nach einem der Ansprüche 1 bis 12 oder einem Instrument (10, 10') nach Anspruch 13, sowie mit einer Versorgungseinrichtung (11), die zur Abgabe einer konstanten oder einer pulsierenden Gleichspannung (Vcc) eingerichtet ist.

15. Einrichtung nach Anspruch 14, **dadurch gekennzeichnet, dass** die Versorgungseinrichtung (11) eine Gleichspannung (Vcc) erzeugend ausgebildet ist, die zwischen einem Minimalwert (Vu) und einem Maximalwert (Vo) alterniert.

## Claims

1. Electrosurgical power generator (22), particularly for creation of a radio frequency voltage (U_{HF}) for treatment of biological tissue,
having a resonant circuit (23) that comprises at least one capacitor (25) and at least one inductor (24) that are connected in parallel to each other in order to form a parallel resonant circuit,
having a first cascode circuit (31) that comprises a first input transistor (33) and a first output transistor (34),
having a second cascode circuit (32) that comprises a second input transistor (35) and a second output transistor (36),
wherein the two output transistors (34, 36) are connected to the resonant circuit (23) and the two input transistors (33, 35) are connected to one another.

2. Power generator according to claim 1, **characterized in that** the first cascode circuit (31) comprises a first input (E1), a first output (A1) and a first intermediate tap (Z1),
that the second cascode circuit (32) comprises a second input (E2), a second output (A2) and a second intermediate tap (Z2),
wherein the outputs (A1, A2) of the cascode circuits (31, 32) are connected to the parallel resonant circuit (23), and
wherein the first intermediate tap (Z1) is connected to the second input (E2) and the second intermediate tap (Z2) is connected to the first input (E1).

3. Power generator according to claim 2, **characterized in that** the inputs (E1, E2) are connected to the intermediate taps (Z2, Z1) in a capacitive manner.

4. Power generator according to any of the preceding claims, **characterized in that** the two output transistors (34, 36) comprise a control electrode (G₃₄, G₃₆) respectively and that the control electrodes (G₃₄, G₃₆) are connected to a direct voltage (V_{G}).

5. Power generator according to any of the preceding claims, **characterized in that** the input transistors (33, 35) are field-effect transistors and respectively comprise a source that is connected to a reference potential (37).

6. Power generator according to claim 5, **characterized in that** the input transistors (33, 35) respectively comprise a drain that is connected to the intermediate tap (Z1, Z2) .

7. Power generator according to claim 5 or 6, **characterized in that** the input transistors (33, 35) respectively comprise a gate as control electrode, wherein the gates are the inputs (E1, E2) of the two cascode circuits (31, 32) .

8. Power generator according to any of the preceding claims, **characterized in that** the output transistors (34, 36) are field-effect transistors.

9. Power generator according to claim 8, **characterized in that** the output transistors (34, 36) respectively comprise a source that is connected to the intermediate tap (Z1, Z2).

10. Power generator according to any of the preceding claims, **characterized in that** the inductor (24) comprises a center tap (26) that is connected to a supply voltage source (V_{cc}).

11. Power generator according to any of the preceding claims, **characterized in that** the inductor (24) is arranged in magnetic coupling with a decoupling inductor (28).

12. Power generator according to claim 9, **characterized in that** the decoupling inductor (28) is connected to at least one electrode (14, 15).

13. Instrument (10, 10') in which a power generator according to any of the preceding claims is arranged.

14. Arrangement having a power generator according to one of the claims 1-12 or an instrument (10, 10') according to claim 13 as well as supply apparatus (11) configured for output of a constant or pulsating direct voltage (V_{cc}).

15. Arrangement according to claim 14, **characterized in that** the supply apparatus (11) is configured for producing a direct voltage (V_{cc}) alternating between a minimum value (Vᵤ) and a maximum value (Vₒ).

## Revendications

1. Générateur de puissance électromédical (22) destiné en particulier à la production d'une tension à haute fréquence (U_{HF}) aux fins de traitement de tissus biologiques,
comprenant un circuit oscillant (23) qui présente au moins un condensateur (25) et au moins une bobine (24) qui sont montés en parallèle l'un par rapport à l'autre pour former un circuit oscillant parallèle,
comprenant premier montage cascode (31) qui présente un premier transistor d'entrée (33) et un premier transistor de sortie (34),
comprenant un deuxième montage cascode (32) qui présente un deuxième transistor d'entrée (35) et un deuxième transistor de sortie (36),
les les deux transistors de sortie (34, 36) étant reliés au circuit oscillant (23) et les deux transistors d'entrée (33, 35) étant reliés entre eux.

2. Générateur de puissance selon la revendication 1, **caractérisé en ce que**
le premier montage cascode (31) présente une première entrée (E1), une première sortie (A1) et une première prise intermédiaire (Z1),
**en ce que** le le deuxième montage cascode (32) présente une deuxième entrée (E2), une deuxième sortie (A2) et une deuxième prise intermédiaire (Z2),
les sorties (A1, A2) des montages cascode (31, 32) étant reliées au circuit oscillant parallèle (23), et
la première prise intermédiaire (Z1) étant reliée à la deuxième entrée (E2), et la deuxième prise intermédiaire (Z2) étant reliée à la première entrée (E1).

3. Générateur de puissance selon la revendication 2, **caractérisé en ce que** les entrées (E1, E2) sont reliées de manière capacitive aux prises intermédiaires (Z2, Z1).

4. Générateur de puissance selon l'une des revendications précédentes, **caractérisé en ce que** les deux transistors de sortie (34, 36) présentent respectivement une électrode de commande (G₃₄, G₃₆), et **en ce que** les électrodes de commande (G₃₄, G₃₆) sont reliées à une tension continue (V_{G}).

5. Générateur de puissance selon l'une des revendications précédentes, **caractérisé en ce que** les transistors d'entrée (33, 35) sont des transistors à effet de champ et présentent respectivement une source qui est reliée à un potentiel de référence (37).

6. Générateur de puissance selon la revendication 5, **caractérisé en ce que** les transistors d'entrée (33, 35) présentent respectivement un drain qui est relié à la prise intermédiaire (Z1, Z2).

7. Générateur de puissance selon la revendication 5 ou 6, **caractérisé en ce que** les transistors d'entrée (33, 35) présentent respectivement une grille en tant qu'électrode de commande, les grilles étant les entrées (E1, E2) des deux montages cascode (31, 32).

8. Générateur de puissance selon l'une des revendications précédentes, **caractérisé en ce que** les transistors de sortie (34, 36) sont des transistors à effet de champ.

9. Générateur de puissance selon la revendication 8, **caractérisé en ce que** les transistors de sortie (34, 36) présentent respectivement une source qui est reliée à la prise intermédiaire (Z1, Z2).

10. Générateur de puissance selon l'une des revendications précédentes, **caractérisé en ce que** la bobine (24) présente une prise médiane (26) qui est reliée à une source de tension d'alimentation (Vcc).

11. Générateur de puissance selon l'une des revendications précédentes, **caractérisé en ce que** la bobine (24) est disposée en couplage magnétique avec une bobine de découplage (28).

12. Générateur de puissance selon la revendication 9, **caractérisé en ce que** la bobine de découplage (28) est reliée à au moins une électrode (14, 15).

13. Instrument (10, 10') dans lequel est disposé le générateur de puissance selon l'une des revendications précédentes.

14. Dispositif comprenant un générateur de puissance selon l'une des revendications 1 à 12 ou un instrument (10, 10') selon la revendication 13, et comprenant un dispositif d'alimentation (11) qui est conçu pour délivrer une tension continue (Vcc) constante ou pulsée.

15. Dispositif selon la revendication 14, **caractérisé en ce que** le dispositif d'alimentation (11) est réalisé de manière à générer une tension continue (Vcc) qui alterne entre une valeur minimale (Vu) et une valeur maximale (Vo).
